# EUROPEAN PATENT APPLICATION

(11) **EP 3 391 827 A1**
(43) Date of publication of application: **24.10.2018**
(21) Application number: 16875671.6
(22) Date of filing: 14.12.2016
(51) Int. Cl.: A61B 10/00

(54) **METHOD AND DEVICE FOR DIAGNOSING SCHIZOPHRENIA**

(30) Priority: 17.12.2015 JP 2015245979
(71) Applicant: Keio University, Tokyo 108-8345 (JP)
(72) Inventor: MAEDA, Takaki, Tokyo 160-8582 (JP)
(74) Representative: Maikowski & Ninnemann Patentanwälte Partnerschaft mbB
(86) International application number: PCT/JP2016/087182
(87) International publication number: WO 2017/104694

(57) **Abstract**

The present invention provides a method and a device for diagnosing schizophrenia. The method of the present invention comprises the steps of: applying a plurality of different sensory stimuli to a subject with any time lag selected from 0 to approximately 1000 msec; obtaining subject's first answer regarding temporal order judgment of the plurality of different sensory stimuli applied; determining whether or not subject's passive time perception is normal on the basis of the first answer; displaying an image of a figure moving at a constant speed; giving a signal to the subject; allowing the subject to respond to the signal, wherein the image of the figure (1) changes due to the subject's response 0 to approximately 2000 msec after the response, or (2) changes irrespective of the subject's response within approximately 1000 msec before or after the signal: and obtaining subject's second answer as to whether or not the change in the image of the figure was caused due to the subject's own response.

## Description

### (Technical Field)

The present invention relates to a method and device for diagnosing schizophrenia. In particular, the present invention relates to a method and device for diagnosing schizophrenia, comprising evaluating sense of agency.

### (Background Art)

Schizophrenia is one of the important psychiatric diseases in psychiatry, because it has a high prevalence, and it is an intractable disease for which no radical treatment method exists. Since the cause of schizophrenia still remains unclear, no method for objectively diagnosing schizophrenia has been established.

Self-disturbance, which is a symptom specific to schizophrenia, is aberrancy in self-consciousness. There has not been any method for scientifically examining self-consciousness until now; however, neuroscientific researches on self-consciousness have been advanced recently, and the aberrance in "sense of agency" and "sense of ownership" has attracted attentions in relation to self-disturbance in schizophrenia. In this paradigm, the self-disturbance in schizophrenia can be empirically evaluated.

Sense of agency has barely been researched in schizophrenia, but an example is the research by Daprati et al., (Non-Patent Literature 1). In this research, a task is used in which a subject is shown a motion of either his/her own hand or a hand of someone else, and instructed to determine the agent of the shown motion of the hand. However, the method has problems such as the point that the temporal bias and the spatial bias are not controlled strictly in the experiment system, the point that the sense of ownership of body is also evaluated simultaneously with the sense of agency, and the point that the experiment device is so large that the method is difficult to implement in ordinary clinical situations and has low versatility.

Accordingly, there is a demand for a novel method and device for diagnosing schizophrenia that enable evaluation of sense of agency, are easy to implement in clinical situations, and have a high versatility.

### (Prior Art)

### (Non-Patent Literature)

Non-Patent Literature 1: Daprati, E., Franck, N., Georgieff, N., Proust, J., Pacherie, E., Dalery, J., Jeannerod, M., 1997. Looking for the agent: an investigation into consciousness of action and self-consciousness in schizophrenic patients. Cognition 65, 71-86.

### (Disclosure of the Invention)

### (Problems to be Solved by the Invention)

In schizophrenia, aberrance in sense of agency is manifested more sharply and strongly, and hence the evaluation of the sense of agency is particularly important. An object of the present invention is to provide a method for diagnosing schizophrenia, a method for acquiring data for diagnosing schizophrenia, and a device therefor which enables evaluation of sense of agency, which are fast and easy to implement in clinical situations, and which have high versatility.

### (Means for Solving the Problems)

In developing a method for diagnosing schizophrenia, the present inventor has examined a task for evaluating sense of agency. Tasks for evaluating sense of agency have been invented in the past, but these tasks are mainly conducted in experimental systems in which, regarding the causality between a voluntary action and an external event resulting from the voluntary action, a comparison is made between subjectivity and objectivity on the temporal axis. In this approach, the physical time in the causality between the voluntary action and the external event is controlled, and a question is made as to the corresponding change in a subjective experience about the agent in response to the manipulation.

However, conventional tasks for evaluating sense of agency have the following problems as described above: (1) the testing method is difficult, and is not suitable for general clinical situations from the standpoints of practicability and invasiveness; (2) since the sense of agency is examined in a body movement, a factor of the sense of ownership is also included; and the like. Accordingly, the sense of agency cannot be evaluated accurately by using any of these tasks. In view of these problems, the present inventor has developed a novel task for evaluating sense of agency which is faster, easier, and less invasive than conventional tasks, and which can be used for severe cases. The task is conducted on a subject, and the subject can be diagnosed as schizophrenia on the basis of the results. Alternatively, the results may help diagnosis schizophrenia in the subject.

Accordingly, the present invention provides a method for diagnosing schizophrenia or a method for acquiring data for diagnosing schizophrenia: the method comprising the steps of:
applying a plurality of different sensory stimuli to a subject with any time lag selected from 0 to approximately 1000 msec;
obtaining subject's first answer regarding temporal order judgment of the plurality of different sensory stimuli applied;
determining whether or not subject's passive time perception is normal on the basis of the first answer, wherein the subject's passive time perception can be determined to be normal when a correct-answer ratio of the first answer is high;
displaying an image of a figure moving at a constant speed to the subject whose passive time perception is determined to be normal in the determination step;
giving a signal to the subject;
allowing the subject to respond to the signal,
wherein the image of the figure either
   (1) changes due to the subject's response 0 to approximately 2000 msec after the response, or
   (2) changes irrespective of the subject's response within approximately 1000 msec before or after the signal; and
obtaining subject's second answer as to whether or not the change in the image of the figure was caused due to the subject's own response;
wherein the subject can be determined to be probably affected with schizophrenia, when an answer ratio of the second answer stating that the change in the image of the figure was caused due to the subject's own response is aberrant in comparison with that of a healthy individual.

The present invention also provides a device for implementing the above-described method. Specifically, the present invention provides a device for diagnosing schizophrenia comprising:
stimulus application means for applying a plurality of different sensory stimuli to a subject with any time lag selected from 0 to approximately 1000 msec;
first answer acquisition means for obtaining subject's first answer regarding temporal order judgment of the plurality of different sensory stimuli applied;
determination means for determining whether or not subject's passive time perception is normal on the basis of the first answer, wherein the subject's passive time perception can be determined to be normal when a correct-answer ratio of the answer is high;
display means for displaying, to the subject whose passive time perception is determined to be normal by the determination means, an image of a figure that moves at a constant speed and that changes at any one of the following times (1) and (2);
signaling means for giving a signal to the subject;
response means for allowing the subject to respond to the signal:
   (1) due to the subject's response 0 to approximately 2000 msec after the response, or (2) irrespective of the subject's response within approximately 1000 msec before or after the signal; and
   second answer acquisition means for obtaining subject's second answer as to whether or not the change in the image of the figure was caused due to the subject's own response,
wherein the subject can be determined to be probably affected with schizophrenia, when an answer ratio of the second answer stating that the change in the image of the figure was caused due to the subject's own response is aberrant in comparison with that of a healthy individual.

The present invention further provides a schizophrenia diagnostic program used for the method and device of the present invention. The schizophrenia diagnostic program of the present invention can cause a computer to execute or operate the method or the device of the present invention. Specifically, the schizophrenia diagnostic program of the present invention is capable of causing a computer to execute the steps of:
instructing the stimulus application means to apply a plurality of different sensory stimuli to a subject with any time lag selected from 0 to approximately 1000 msec;
acquiring, from the first answering means, subject's first answer regarding temporal order judgment of the plurality of different sensory stimuli applied;
calculating a correct-answer ratio of the first answer on the basis of the first answer, and outputting a result of a determination that subject's passive time perception is normal, when the correct-answer ratio is high;
causing the display means to display an image of a figure moving at a constant speed to the subject whose passive time perception is determined to be normal;
instructing the signaling means to give a signal to the subject;
acquiring subject's response to the signal from the response means;
causing the image of the figure on the display means to
   (1) change due to the subject's response 0 to approximately 2000 msec after the response, or
   (2) change irrespective of the subject's response within approximately 1000 msec before or after the signal;
acquiring, from the second answering means, subject's second answer as to whether or not the change in the image of the figure was caused due to the subject's own response; and
calculating an answer ratio of the answer stating that the change in the image of the figure was caused due to the subject's own response based on the second answer, and outputting a result of a determination that the subject is probably affected with schizophrenia, when the answer ratio is aberrant in comparison with that of a healthy individual. The present invention further provides a computer-readable recording medium that records the schizophrenia diagnostic program of the present invention.

In one embodiment, the plurality of different sensory stimuli are a tactile stimulus and a visual stimulus. In a specific embodiment, the plurality of different sensory stimuli are a tactile stimulus and a visual stimulus, and the tactile stimulus and the visual stimulus are applied in order and with a time lag according to any one of the following (i) to (v): (i) the tactile stimulus, and the visual stimulus approximately 50 msec after the tactile stimulus; (ii) the tactile stimulus, and the visual stimulus approximately 100 msec after the tactile stimulus; (iii) the tactile stimulus and the visual stimulus simultaneously; (iv) the visual stimulus, and the tactile stimulus approximately 50 msec after the visual stimulus; or (v) the visual stimulus, and the tactile stimulus approximately 100 msec after the visual stimulus. In a further specific embodiment, the tactile stimulus is a vibration stimulus, and the visual stimulus is a light stimulus.

In one embodiment, the change in the image of the figure is disappearance and appearance of the figure (the subject has an experience, as if the figure made a jump). In one embodiment, the image of the figure (1) changes due to the subject's response 0 to approximately 1000 msec after the response, or (2) changes irrespective of the subject's response within approximately 100 msec before and after the signal.

In one embodiment, the image of the figure moving at a constant speed is displayed by turning on a light of one of two cursors on a belt portion moving at a constant speed. Here, the change in the image of the figure is caused by switching the turning-on of the light to turning-on of a light of the other cursor.

### (Effects of the Invention)

The method and device of the present invention enable evaluation of subject's sense of agency. The method and device of the present invention is easier to implement and less invasive than conventional methods and devices, and can also be used for severe cases.

### (Brief Description of the Drawings)

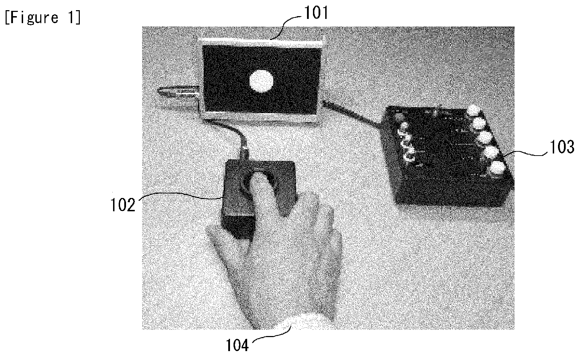

Figure 1 is a specific example of stimulus application means for applying a tactile stimulus and a visual stimulus to a subject. The stimulus application means of Figure 1 comprises a display (101) that applies a visual stimulus, a vibrator (102) that applies a tactile stimulus, and a controller (103) that controls these stimuli. Here, the tactile stimulus and the visual stimulus are a vibration stimulus and a light stimulus, respectively. A subject (104) places his/her finger on the vibrator (102). The subject can receive a tactile stimulus, when the vibrator (102) vibrates.

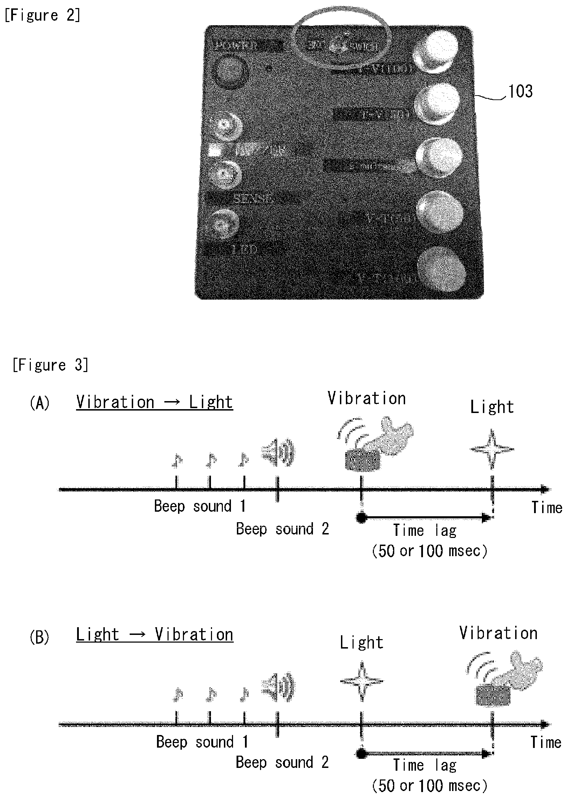

Figure 2 shows a specific example of a controller for controlling a sensory stimulus applied to a subject. The controller can control the order of and the time lag between a tactile stimulus and a visual stimulus, and the like. The controller has two modes: "SWITCH mode" and "BNC mode." In the "SWITCH mode," predetermined stimuli can be applied by pressing any one of buttons on the right side of the controller. In the "BNC mode," corresponding stimuli can be applied by inputting a TTL signal (5 V) to a BNC connector.
Figure 3 shows examples of a case (Figure 3A) where "T-V(100)" or "T-V(50)" is pressed, and a case (Figure 3B) where "V-T(50)" or "V-T(100)" is pressed, with the controller (103) of Figure 2 set in the "SWITCH mode."

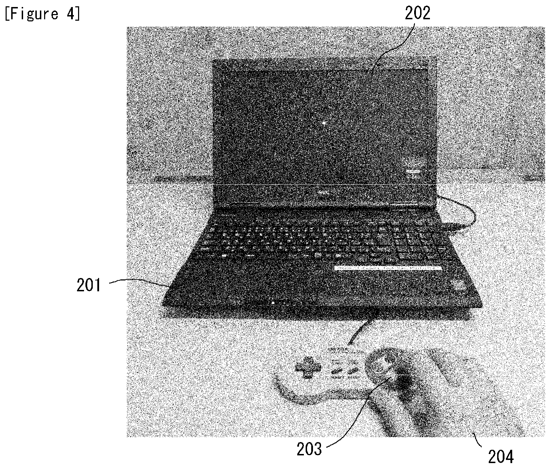

Figure 4 shows an example of a device for implementing a sense-of-agency test. The device of Figure 4 comprises a computer (201) that controls an image of a figure, a display (202) that displays the image of the figure, and a button (203) for a subject to respond to the signal.

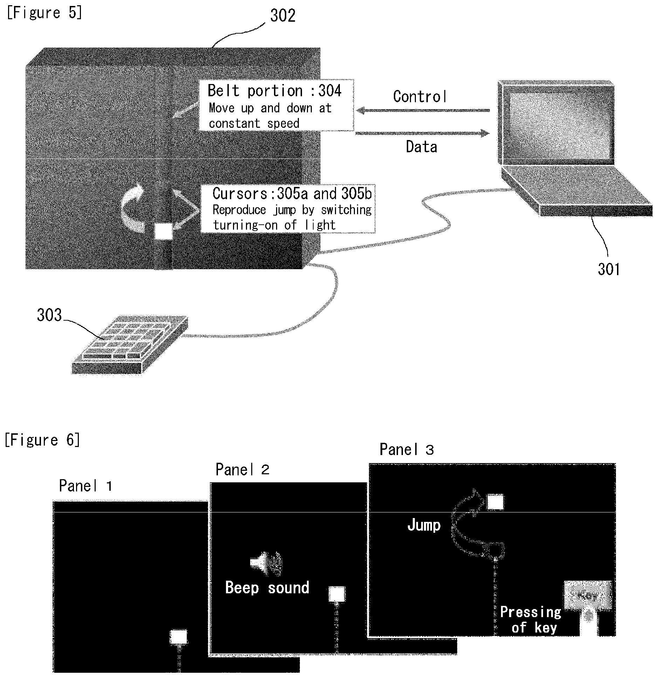

Figure 5 is another example of a device for conducting the sense-of-agency test. The device of Figure 5 comprises a computer (301) that controls an image of a figure, an external display (302) that displays the image of the figure, and a response key (303) for a subject to respond to the signal. The external display (302) comprises a belt portion (304) and two cursors (305a and 305b).
Figure 6 shows a specific example of the change in the image of the figure. An quadrangle image moves at a constant speed on the display (Panel 1), a subject presses a key in response to a beep sound signal (Panel 2), and the image of the figure on the display makes a jump (Panel 3).

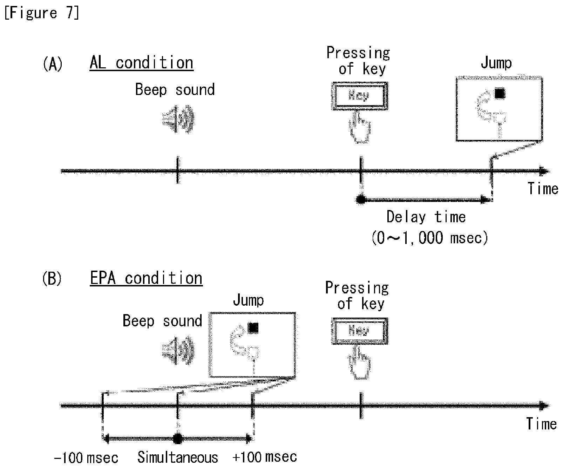

Figure 7 shows a flow from the application of the signal to a subject, through the response from the subject to the signal, to the change in the image. A condition (AL condition) under which the image of the figure changes due to the subject's response (Figure 7A). A condition (EPA condition) under which the image of the figure changes irrespective of the subject's response (Figure 7B).

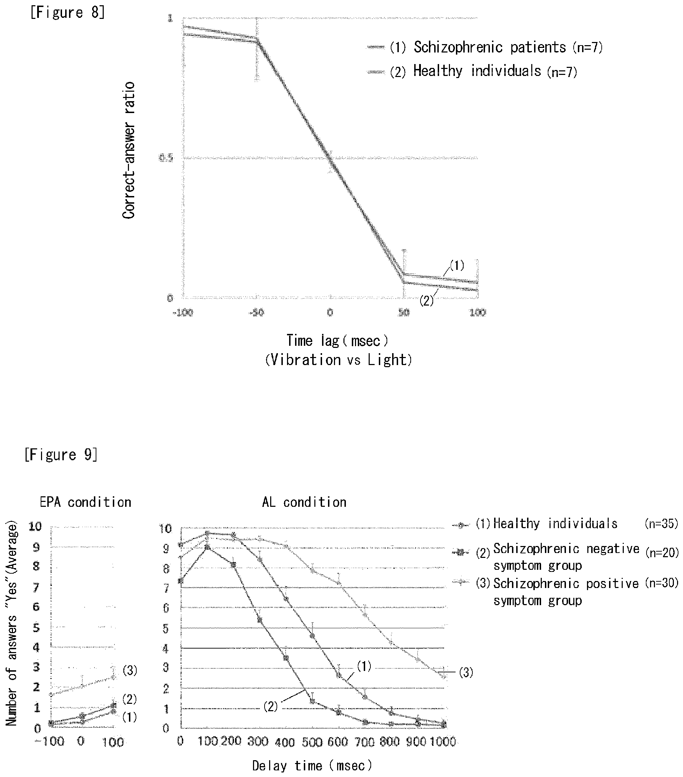

Figure 8 shows the results of the passive time perception test of the present invention conducted on healthy individuals and schizophrenic patients. In the graph, the horizontal axis represents the time lag (msec) between two sensory stimuli (a vibration stimulus and a light stimulus). On the horizontal axis, 100 means that a vibration stimulus is applied, and a light stimulus is then applied 100 msec after the vibration stimulus, and - 100 means that a light stimulus is applied, and a vibration stimulus is then applied 100 msec after the light stimulus. In the graph, the vertical axis represents the ratio of answers stating that the two sensory stimuli were in the order of the vibration stimulus and the light stimulus, which were obtained when the test was conducted 10 times under each condition.
Figure 9 shows the results of the sense-of-agency test of the present invention conducted on healthy individuals, a schizophrenic negative symptom group, and a chronic schizophrenic positive symptom group. The results in the case of the AL condition (right graph). The results in the case of the EPA condition (left graph). The horizontal axis represents the time at which the change of the image occurred, and the vertical axis represents the number of times of answering with "Yes" to the question that "Did you feel that your pressing of the key caused the figure to make a jump?".

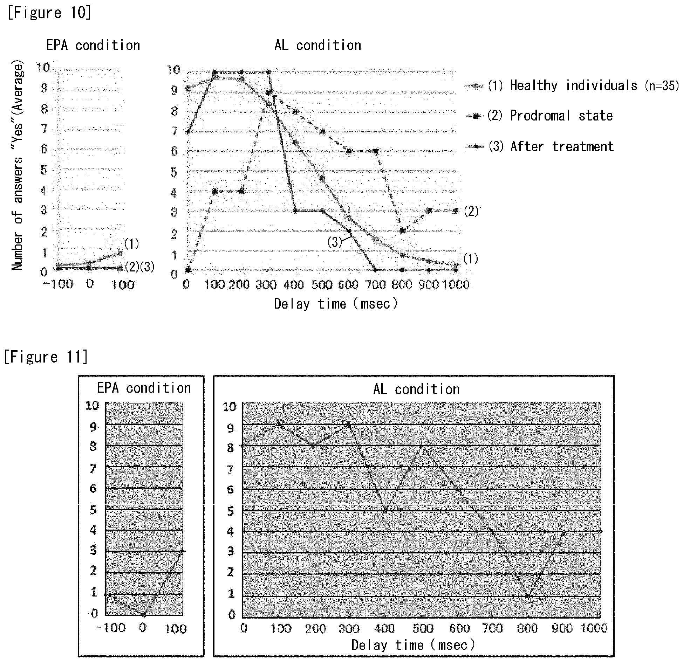

Figure 10 shows the results of the sense-of-agency test of the present invention conducted on healthy individuals, patients in a prodromal state before onset of schizophrenia, and treated patients. The horizontal axis and the vertical axis are the same as those in Figure 9.
Figure 11 shows the results of the sense-of-agency test of the present invention conducted on patients at an acute stage who had actually developed schizophrenia. The horizontal axis and the vertical axis are the same as those in Figure 9.

### (Description of Embodiments)

### (Definition)

As used herein, "sense of agency" has a meaning commonly known by those skilled in the art, and refers to a sense that a person, him/herself, is the agent of an action or thought, i.e., a subjective experience that a person can control him/her own body movement or external events by him/herself. The "sense of agency" is also referred to as "sense of self-agency."

As used herein, "sense of ownership of body," on which the sense of agency is based, is a sense that the body, action, and thought of a person are owned by him/herself.

As used herein, "passive time perception" has a meaning commonly used by those skilled in the art, and means a simple passive perception such as a lapse of time, the length of time, and/or the temporal before-and-after relationship regarding a given phenomenon.

As used herein, a "subject" means a person subjected to the method for diagnosing schizophrenia of the present invention. The subject is, for example, a healthy individual, a schizophrenic patient, a patient with suspected schizophrenia, a patient diagnosed with non-schizophrenia, a patient with other neuropsychiatric disease, or the like. In the description of the present application, a person who implements the method for diagnosing schizophrenia of the present invention on a "subject" is described as a "rater."

As used herein, " approximately" refers to a range or an error range acceptable by those skilled in the art, and indicates that variation by, for example, 0.1 to 10%, 0.1 to 5%, 0.1 to 1.0%, or 0.1 to 0.5% may exist.

For convenience of description, subject's answer regarding temporal order judgment of a plurality of different sensory stimuli applied may also be described herein as a "first answer." Answer acquisition means for acquiring the "first answer" may also be described as "first answer acquisition means."

For convenience of description, the subject's answer as to whether or not a change in an image of a figure was caused due to the subject's own response may also described herein as a "second answer." Answer acquisition means for acquiring the "second answer" may be also described as "second answer acquisition means."

### (Methods and Device for Diagnosing Schizophrenia of the Present Invention)

The present invention provides a method for diagnosing schizophrenia and a method for acquiring data for diagnosing schizophrenia of the present invention. Specifically, the methods of the present invention comprise a passive time perception test and a sense-of-agency test. Subject's passive time perception can be assessed by the passive time perception test. Subject's sense of agency can be assessed by the sense-of-agency test. A combination of these tests enables diagnosis of subject's schizophrenia. The present invention also provides a device or system for implementing the methods of the present invention. The system can comprise a device for conducting the passive time perception test and a device for conducting the sense-of-agency test. The present invention further provides a program for causing a computer to execute the steps in the methods and operate the means in the device or system of the present invention. All or some of the means in the device or system may be connected to the computer. The program may be recorded in a recorded computer-readable recording medium, such as a CD, a DVD, or a USB memory.

### (Passive Time Perception Test of the Present Invention)

The passive time perception test of the present invention is a test for determining whether or not a subject has normal passive time perception by imposing a temporal order judgment task (Keio method) on the subject. The sense-of-agency test of the present invention assumes that the subject has normal passive time perception. Hence, if the subject does not have normal passive time perception, the reliability of the results of the sense-of-agency test of the present invention would be low. The passive time perception test guarantees the reliability of interpretation of the results of the sense-of-agency test.

The passive time perception test of the present invention comprises the steps of: (a) applying a plurality of different sensory stimuli to a subject with any time lag selected from 0 to approximately 1000 msec; and (b) obtaining subject's first answer regarding temporal order judgment of the plurality of different sensory stimuli applied. The passive time perception test of the present invention can further comprise the step of (c) determining whether or not subject's passive time perception is normal on the basis of the first answer, wherein the subject's passive time perception can be determined to be normal, when a correct-answer ratio of the first answer is high.

In step (a), the sensory stimulus is a noninvasive stimulus. Examples of the sensory stimulus include a tactile stimulus, a visual stimulus, an auditory stimulus, and the like. The plurality of sensory stimuli are, for example, two, three, four, or more sensory stimuli. Preferably, the plurality of sensory stimuli are two sensory stimuli. Examples of combinations of plurality of different sensory stimuli include one tactile stimulus with one visual stimulus, one tactile stimulus with one auditory stimulus, one visual stimulus with one auditory stimulus, two different tactile stimuli, two different visual stimuli, two different auditory stimuli, and the like. Preferably, the plurality of different sensory stimuli are a tactile stimulus and a visual stimulus. Examples of the visual stimuli include light stimuli and color stimuli such as light flash and change in color. Examples of the tactile stimuli include a vibration stimulus, a pressure stimulus, a thermal stimulus, and the like. Examples of the auditory stimuli include sounds such as a beep sound. In the passive time perception test of the present invention, the plurality of different sensory stimuli are preferably a tactile stimulus and a visual stimulus, and more preferably a vibration stimulus and a light stimulus, considering the consistency with the sense-of-agency test.

In step (a), the plurality of different sensory stimuli are applied with any time lag selected from 0 to approximately 1000 msec. For example, the plurality of different sensory stimuli are applied with a time lag of 0, approximately 50 msec, approximately 100 msec, approximately 200 msec, approximately 300 msec, approximately 400 msec, approximately 500 msec, approximately 600 msec, approximately 700 msec, approximately 800 msec, approximately 900 msec, or approximately 1000 msec. The time lag "0" means that the plurality of different sensory stimuli are applied simultaneously. Preferably, the plurality of different sensory stimuli are applied with a time lag of 0 to approximately 100 msec. When the passive time perception test is conducted more precisely, the time lags such as approximately 25 msec or approximately 75 msec should be introduced; however, the time lags should be determined considering the invasiveness to the subject.

In step (a), the plurality of different sensory stimuli are preferably a tactile stimulus and a visual stimulus, and the stimuli are applied in order and with a time lag according to any one of the following (i) to (v): (i) the tactile stimulus, and the visual stimulus approximately 50 msec after the tactile stimulus; (ii) the tactile stimulus, and the visual stimulus approximately 100 msec after the tactile stimulus; (iii) the tactile stimulus and the visual stimulus simultaneously; (iv) the visual stimulus, and the tactile stimulus approximately 50 msec after the visual stimulus; or (v) the visual stimulus, and the tactile stimulus approximately 100 msec after the visual stimulus.

The rater can randomly select the types of, the order of, and the time lag between the plurality of different sensory stimuli, and apply them to the subject. In addition, the types of, the order of, and the time lag between the plurality of different sensory stimuli may be selected at random by a computer.

Prior to step (a), a step of applying a signal to the subject may be included. The subject can know the timing of application of the sensory stimuli from the signal. Examples of the signal include sounds such as a beep sound, and vibration.

Step (a) can be conducted by using stimulus application means for applying a plurality of different sensory stimuli with any time lag selected from 0 to approximately 1000 msec. Figure 1 shows an example of the stimulus application means. In Figure 1, the stimulus application means comprises a display (101) that applies a visual stimulus, a vibrator (102) that applies a tactile stimulus, and a controller (103) that controls these stimuli. Here, the tactile stimulus and the visual stimulus are a vibration stimulus and a light stimulus (light flash), respectively. The display (101) that applies a visual stimulus has a loudspeaker (not-illustrated) capable of emitting a beep sound on the back side. A step of applying stimuli to a subject by using the stimulus application means of Figure 1 is described below.

The subject (104) keeps his/her finger placed on the vibrator (102). The rater operates the controller (103) to present a light (light flash) on the display (101) for the subject and vibrate the vibrator (102). In addition, before application of the sensory stimulus, a beep sound can be emitted from the loudspeaker on the back of the display (101) to give the subject a signal that the sensory stimuli are about to be applied. Figure 2 shows a specific example of the controller (103).

The controller (103) can control the order of and the time lag between the tactile stimulus and the visual stimulus, and the like. By flicking a switch present in the circled portion in Figure 2, the stimuli can be applied to the subject in one of the two modes: "SWITCH mode" and "BNC mode." In the "SWITCH mode," predetermined stimuli can be applied with a specific time lag by pressing any one of five buttons on the right side of the controller. In the "BNC mode," corresponding stimuli can be applied by inputting a TTL signal (5 V) to a BNC connector.

The five buttons on the right side of the controller (103): "T-V(100)", "T-V(50)", "simultaneous", "V-T(50)", and "V-T(100)" from the top correspond to "a vibration stimulus, and a light stimulus 100 msec after the vibration stimulus," "a vibration stimulus, and a light stimulus 50 msec after the vibration stimulus," "a vibration stimulus and a light stimulus simultaneously," "a light stimulus, and a vibration stimulus 50 msec after the light stimulus," and "a light stimulus, and a vibration stimulus 100 msec after the light stimulus," respectively.

Specifically, in the "SWITCH mode," the rater presses any one of the five buttons on the right side. Then, beep sounds are emitted in the order of beep sound 1 (first time), waiting for 950 msec, beep sound 1 (second time), waiting for 950 msec, beep sound 1 (third time), waiting for 950 msec, and beep sound 2. The beep sound 2 is a sound different from beep sound 1. The sensory stimuli corresponding to any one of the buttons pressed by the rater can be applied to the subject 50 msec after beep sound 2.

In the "BNC mode," when a TTL (Transistor-Transistor Logic) signal is inputted to a BUZZER terminal, beep sounds are emitted in the order of beep sound 1 (first time), waiting for 950 msec, beep sound 1 (second time), waiting for 950 msec, beep sound 1 (third time), waiting for 950 msec, and beep sound 2. When a TTL signal is inputted to a SENSE terminal, a vibration stimulus and a light stimulus 100 msec after the vibration stimulus are applied to the subject. When a TTL signal is inputted to an LED terminal, a light stimulus and a vibration stimulus 50 msec after the light stimulus are applied to the subject.

An example of the step of applying stimuli to a subject by using the stimulus application means shown in Figure 1 is described based on Figures 2 and 3. The rater sets the controller (103) of Figure 2 in the "SWITCH mode," and presses the button of "T-V(100)" or "T-V(50)." Beep sound 1 is emitted three times, and beep sound 2 is emitted once (Figure 3A). A tactile stimulus (vibration stimulus) is applied to the subject 50 msec after beep sound 2 is emitted, and a visual stimulus (light stimulus) is applied 50 msec or 100 msec after the tactile stimulus. Meanwhile, the rater sets the controller (103) of Figure 2 in the "SWITCH mode," and presses the button of "V-T(50)" or "V-T(100)." Beep sound 1 is emitted three times, and beep sound 2 is emitted once (Figure 3B). A visual stimulus (light stimulus) is applied to the subject 50 msec after beep sound 2 is emitted, and a tactile stimulus (vibration stimulus) is applied 50 msec or 100 msec after the visual stimulus.

In step (b), the subject answers the order of the sensory stimuli applied. For example, the subject answers a question such as "which was applied first?", "which was applied later?", or "in what order the stimuli were applied?" regarding the plurality of different sensory stimuli applied, for example, two different sensory stimuli. The method for the answering may be, for example, a method in which answering buttons are provided in advance, and the subject is instructed to press an answering button. In the present description, this answer is also referred to as a "first answer." The first answer is obtained, and whether or not the answer is correct can be judged. The first answer can be obtained by suitable answer acquisition means. In the present description, the answer acquisition means is also referred to as "first answer acquisition means." The first answer acquisition means can comprise answer input means such as answering buttons.

Step (a) and step (b) can be performed repeatedly on the subject. The time taken to conduct step (a) and step (b) once is several seconds to several tens of seconds. Step (a) and step (b) can be performed repeatedly to obtain multiple subject's first answers regarding temporal order judgment of the sensory stimuli. When step (a) and step (b) are performed repeatedly, the types of and/or the time lag between the sensory stimuli can be changed variously. The number of times of the repetition under each condition can be approximately 10, approximately 20, approximately 30, approximately 40, or approximately 50, or more. Preferably, the number of times of the repetition is 10 under each condition, and 50 in total.

On the basis of the subject's first answers in step (c), whether subject's passive time perception is normal can be determined. Specifically, when the correct-answer ratio of the subject's first answers is high, the subject's passive time perception can be determined to be normal. The correct-answer ratio can be calculated for each condition by (the number of correct answers)/(the number of times of performing steps (a) to (b))×100. For example, when the correct-answer ratio of the subject's first answer is approximately 60% or higher, approximately 70% or higher, approximately 80% or higher, approximately 90% or higher, approximately 95% or higher, or approximately 100%, the subject's passive time perception can be determined to be normal. The determination may be made immediately after the first answers are obtained, or only the correct-answer ratio is first calculated from the first answers, and the determination may be made, for example, after completion of all the tests of the present invention.

It is conceivable that a subject having normal passive time perception will achieve a higher correct-answer ratio when the time lag between the plurality of different sensory stimuli are longer, and achieve a lower correct-answer ratio when the time lag is shorter. Accordingly, the correct-answer ratio may be calculated on the basis of only the answers for the cases where the time lag between the plurality of different sensory stimuli are long to determine whether or not the subject has normal passive time perception. For example, the correct-answer ratio is calculated on the basis of the answers for the cases where the time lags between the plurality of different sensory stimuli are approximately 50 msec or longer. When the correct-answer ratio is approximately 80% or higher, approximately 90% or higher, approximately 95% or higher, or approximately 100%, the subject's passive time perception can be determined to be normal. Alternatively, for example, the correct-answer ratios are calculated for the cases where the time lags between the plurality of different sensory stimuli are approximately 50 msec and approximately 100 msec. When the correct-answer ratios for the case of approximately 50 msec and the case of approximately 100 msec are approximately 80% or higher and approximately 90% or higher, respectively, the subject's normal passive time perception may be determined to be normal. A statistically desirable judgement criteria is that the subject's normal passive time perception is determined to be normal, when the correct-answer ratio falls within the range of ±1 or ±2SD (standard deviation) from data accumulated from healthy individuals.

Whether or not the passive time perception is normal can be judged by using suitable determination means. The determination means is, for example, a computer programed to be capable of determining whether or not each of the first answers obtained from a subject is correct, and calculating the correct-answer ratio. The computer may be programed to determine that the passive perception is normal, when the correct-answer ratio of the first answers is not lower than a specific percentage, or falls within ±1 or 2SD (standard deviation) from data from healthy individuals. The determination means can comprise means for judging whether a first answer is correct, and/or means for calculating the correct-answer ratio.

### (Sense-of-Agency Test of the Present Invention)

The sense-of-agency test of the present invention is a test in which a sense-of-agency task (Keio method) is imposed on a subject to determine whether or not subject's sense of agency is normal. The sense-of-agency test of the present invention can be conducted on a subject determined to have normal passive time perception by the passive time perception test. The sense-of-agency test of the present invention assumes that the subject has normal passive time perception. Hence, if the subject does not have normal passive time perception, the reliability of the results of the sense-of-agency test of the present invention would be low.

The sense-of-agency test of the present invention comprises the steps of: (d) displaying an image of a figure moving at a constant speed to a subject, (e) giving a signal to the subject, and (f) allowing the subject to respond to the signal. Here, the image of the figure changes due to the subject's response 0 to approximately 2000 msec after the response (condition (1)), or changes irrespective of the subject's response within approximately 1000 msec before or after the signal (condition (2)). The sense-of-agency test of the present invention further comprises the step of (g) obtaining subject's second answer as to whether or not the change in the image of the figure was caused due to the subject's own response.

In step (d), the image of the figure moving at a constant speed is displayed on suitable display means such as a display, a monitor, or a screen. The image of the figure is not particularly limited, and may be a figure such as a quadrangle, a circle, a dot, a triangle, or a diamond. Preferably, the image of the figure is a quadrangle. The color of the image of the figure may be, but is not particularly limited to, white, black, red, blue, yellow, green, or the like. The size of the quadrangle is, for example, 3 mm, 4 mm, 5 mm, 6 mm, or 7 mm square. A preferred size of the quadrangle is 5 mm square. The speed at which the image of the figure moves is not particularly limited, as long as healthy individuals can keep sight of the image moving at that speed. For example, the speed at which the image of the figure moves is approximately 10 to approximately 30 mm/second, and is, for example, approximately 10, approximately 20, or approximately 30 mm/second. From the heuristic standpoint, the speed is preferably approximately 22 mm/second. The moving direction is not particularly limited. For example, the moving direction is from the bottom to the top, from the top to the bottom, from the right to the left, or from the left to the right. Preferably, the moving direction is from the bottom to the top. The image of the figure on the display means changes due to the subject's response in step (f) 0 to approximately 2000 msec after the response (condition (1)), or changes irrespective of the subject's response within approximately 1000 msec before or after the signal in step (e) (condition (2)). The conditions (1) and (2) are described in further detail below.

Step (d) can comprise the step of turning on a light of one of two cursors on a belt portion moving at a constant speed. By turning on the light of the cursor, an image of a figure can be displayed. In some embodiments, the display means for displaying an image of a figure can comprise a belt portion moving at a constant speed and two cursors joined to the belt portion. The cursors may be a lighting portion such as a light-emitting diode (LED). By turning on the light of one of the two cursors, the image of the figure is displayed on the display means such as a display. The change in the image of the figure can be expressed by turning off the light of the cursor of which the light has been turned on and turning on a light of the other cursor, in other words, by switching the turning-on of the light of the one cursor to turning-on of a light of the other cursor.

In step (e), the signal to the subject may be, but not particularly limited to, an auditory stimulus (for example, a sound such as a beep sound), a tactile stimulus (for example, a vibration stimulus), a visual stimulus (for example, a light stimulus such as light flash), or the like. These stimuli are, of course, noninvasive stimuli. Preferably, the signal to the subject in step (e) is a sound such as a beep sound. The signal to the subject can be applied by suitable signaling means capable of applying an auditory stimulus, a tactile stimulus, a visual stimulus, and the like to a subject. For example, the signaling means used may be the same as the stimulus application means used in the passive time perception test of the present invention, and, for example, it is possible to use the loudspeaker on the back side of the display (101) that applies a visual stimulus.

In step (f), the subject responds to the signal in step (e). For the response, the subject only needs to take any action to the signal. The rater can obtain the subject's response to the signal in step (e). For example, a response button or a key may be provided in advance, and the subject may be instructed to press the response button or key. The response can be obtained by suitable response means. The response means can comprise response input means such as a response button or key. Response measurement data such as the time from the signal to the response may be recorded. These data may be used to judge whether or not the subject's sense of agency is normal.

The image of the figure displayed in step (d) can change under the following condition (1) or (2). Condition (1): the image of the figure changes due to the subject's response 0 to approximately 2000 msec after the response; or condition (2): the image of the figure changes irrespective of the subject's response within approximately 1000 msec before or after the signal. In the present description, condition (1) is also referred to as AL condition (Action-linked condition), and condition (2) is also referred to as an EPA condition (Event prior to action condition). The display means for displaying an image of a figure can be connected to a computer or a controller for controlling the speed of the image of the figure, the moving direction of the image of the figure, condition (1) and condition (2), and/or the time at which the image changes, and the like.

In the case of the AL condition, the image of the figure changes simultaneously with the subject's response or after the subject's response. The rater can select the delay time at random and set the delay time. For example, the image of the figure changes 0 to approximately 2000 msec after or 0 to approximately 1000 msec after, for example, 0, approximately 100, approximately 200, approximately 300, approximately 400, approximately 500, approximately 600, approximately 700, approximately 800, approximately 900, or approximately 1000 msec after the subject's response. Here, the time "0" means that the response and the change are simultaneous.

Under the EPA condition, the image of the figure changes within approximately 1000 msec before or after, and preferably within approximately 100 msec before or after the time point of the signal, which is employed as a reference. For example, the image of the figure changes 1000 msec before the signal, 900 msec before the signal, 800 msec before the signal, 700 msec before the signal, 600 msec before the signal, 500 msec before the signal, 400 msec before the signal, 300 msec before the signal, 200 msec before the signal, 100 msec before the signal, simultaneously with the signal, 100 msec after the signal, 200 msec after the signal, 300 msec after the signal, 400 msec after the signal, 500 msec after the signal, 600 msec after the signal, 700 msec after the signal, 800 msec after the signal, 900 msec after the signal, or 1000 msec after the signal.

The rater can select at random the AL condition or the EPA condition, and the time at which the change in the image of the figure occurs. Alternatively, they may be selected by a computer at random.

The change in the image of the figure is not particularly limited, as long as the subject can visually recognize the change. The change in the image of the figure is, for example, disappearance of the figure, disappearance and appearance of the figure, change to another figure, or the like. Preferably, the change in the image of the figure is disappearance and appearance of the figure. For example, the figure moving at a constant speed disappears, and appears at a different position in the same advancing direction. The figure that has appeared can then keep moving further at the same speed. In this case, the image can be seen by the subject, as if the figure made a jump. Preferably, the figure in the image makes a jump by a distance of approximately 25 mm to approximately 45 mm, or approximately 30 mm to approximately 40 mm, and preferably approximately 35 mm.

The sense-of-agency test of the present invention comprises the step of obtaining subject's answer as to whether or not the change in the image of the figure in step (g) was caused due to the subject's own response. The answer may be either "Yes" or "No." For example, it is possible to provide answering buttons in advance, and instruct the subject to press the corresponding answering button. In the present description, the answer is also referred to as a "second answer." Based on the second answer, the subject's sense of agency can be assessed. The second answer can be obtained by suitable answer acquisition means. In the present description, the answer acquisition means is also referred to as "second answer acquisition means." The second answer acquisition means can comprise answer input means such as answering buttons.

Steps (d) to (g) can be performed repeatedly on the subject. The time taken to conduct steps (d) to (g) once may be several seconds to several tens of seconds. Steps (d) to (g) can be performed repeatedly to obtain multiple subject's second answers. When steps (d) to (g) are performed repeatedly, the AL condition, the EPA condition, and the time at which the image changes can be changed variously. The number of times of the repetition under each condition can be approximately 10, approximately 20, approximately 30, or more. Preferably, steps (d) to (g) are repeated every 100 msec 10 times under each condition, and 140 times in total (under 11 AL conditions and 3 EPA conditions).

Figure 4 shows an example of a device for conducting the sense-of-agency test. The device of Figure 4 comprises a computer (201) and a response button (203). The computer (201) has a display (202). The computer (201) and the response button (203) may be connected to each other through a USB cable or the like. The computer (201) can have a built-in loudspeaker capable of emitting a beep sound for signaling. An image of a figure moving at a constant speed is displayed on the display (202). The computer (201) can control the displaying of the image of the figure, the AL condition, the EPA condition, the time at which the image of the figure changes, the number of times of the repetition, and the like

A sense-of-agency test using the device of Figure 4 can be conducted, for example, as follows. The subject (204) keeps his/her finger placed on the response button (203). An image of a figure (a white quadrangle figure) moving at a constant speed is displayed on the display (202). The built-in loudspeaker of the computer (201) emits a beep sound. Upon hearing the beep sound, the subject presses the response button (203). In the case of the AL condition, the image of a figure on the display (202) changes due to the subject' pressing of the response button (203). In the case of the EPA condition, the image of the figure on the display changes before or after the beep sound irrespective of whether or not the subject pressed the response button (203).

In general, the image processing on the display is conducted by a computer. When the image processing is conducted by a computer, a time lag of approximately 35 msec to approximately 100 msec occurs even under a condition essentially free of a time delay, because of the processing of the computer. In such a case, the time lag for the image processing is desirably constant. An acceptable range of the time lag is from 0 to approximately 100 msec, and the time lag is preferably approximately 35 msec. A program for the sense-of-agency test can have a function of calibrating the time lag. However, some computers may have difficulty in calibrating the time lag or may have great fluctuations. For this reason, a display is also necessary which is capable of adjusting the time lag with a precision of approximately 1 msec. Figure 5 shows an example of a device for conducting the sense-of-agency test comprising such a display.

The device of Figure 5 comprises a computer (301), an external display (302), and a response key (303). The computer (301), the external display (302), and the response key (303) may be connected together through USB cables or the like. The computer (301) or the external display (302) can comprise a built-in loudspeaker capable of emitting a beep sound for signaling. The external display (302) comprises a belt portion (304) at a central portion thereof. To the belt portion (304), two cursors (305a and 305b) (for example, light-emitting diodes: LEDs) are fixed. The two cursors (305a and 305b) may be configured to display an image of a figure such as a quadrangle figure on the external display (302), when each cursor is turned on. The distance between the two cursors (305a and 305b) is approximately 25 mm to approximately 45 mm, or approximately 30 mm to approximately 40 mm, and preferably is approximately 35 mm. The belt portion (304) is moved up and down by a motor or the like, and can move the two cursors (305a and 305b) up and down. The speed at which the two cursors (305a and 305b) move is approximately 10 to approximately 30 mm/second, and is, for example, approximately 10, approximately 20, or approximately 30 mm/second, and preferably approximately 22 mm/second. A jump can be reproduced by switching the turning-on of the light between the two cursors (305a and 305b). The computer (301) can control the turning-on and turning-off of the light of the two cursors (305a and 305b), the AL condition ,the EPA condition, the time at which the switching of the turning-on of the light between the two cursors (305a and 305b) is started, the number of times of the repetition, and the like.

A sense-of-agency test using the device of Figure 5 is conducted, for example, as follows. Here, the lower one of the two cursors in Figure 5 is referred to as a cursor (305a), and the upper one is referred to as a cursor (305b). The subject keeps his/her finger placed on the response key (303). The rater operates the computer (301) to turn on the light of the cursor (305a). An image of a figure is displayed on the external display (302). The light of the cursor (305b) remains turned off. The belt portion (304) is moved upward from the bottom at a constant speed. This moves the two cursors (305a and 305b) from the bottom to the top at the constant speed, so that an image of a figure moving at the constant speed is displayed on the external display (302). The built-in loudspeaker of the external display (302) emits a beep sound. Upon hearing the beep sound, the subject presses the response key (303). In the case of the AL condition, the light of the cursor (305a) is tuned off and the light of the cursor (305b) is turned on due to the subject's pressing of the response key (303). In the case of the EPA condition, the light of the cursor (305a) is tuned off, and the light of the cursor (305b) is turned on before or after the beep sound, irrespective of whether or not the subject presses the response key (303). By switching the turning-on of the light between the two cursors (305a and 305b), the image can be seen by the subject, as if the figure made a jump.

In the device of Figure 5, subject's response measurement data and the like, for example, the time between the beep sound and the pressing of the response key (303) can be recorded in the computer (301). These data may be used to judge whether or not the subject's sense of agency is normal. The same shall apply to the device of Figure 4.

Specific examples of the displaying of the image of the figure, the signal, the subject's response to the signal, the change in the image of the figure, and the subject's second answer in steps (d) to (g) are described in further detail on the basis of Figures 6 and 7. Referring to Figure 6, a quadrangle figure on the display is advancing from the bottom to the top at a constant speed (Panel 1). After a beep sound signal (Panel 2), the subject presses the key. The quadrangle figure disappears from the display, and appears at a different position in the same advancing direction (Panel 3). The quadrangle figure is seen, as if it made a jump.

Figure 7 shows examples of the change in the image of the figure under the AL condition (Figure 7A) and the EPA condition (Figure 7B). In the case of the AL condition, after the beep sound signal, the subject presses the key in response to the signal. The quadrangle figure makes a jump 0 to 1000 msec after the subject presses the key. In the case of the EPA condition, in contrast, the image of the figure makes a jump 100 msec before the beep sound signal, simultaneously with the beep sound signal, or 100 msec after the beep sound signal. Upon hearing the beep sound, the subject presses the key. No change occurs in the quadrangle figure due to the pressing of the key. The subject answers a question as to whether or not the jump of the quadrangle figure was caused due to the pressing of the key.

Based on the second answer in step (g), it is determined whether or not the subject's sense of agency is normal, and subject's schizophrenia can be diagnosed. The present inventor has found that the sense of agency of schizophrenic patients is aberrant in comparison with that of healthy individuals. Specifically, a schizophrenic patient may have a stronger sense that the change in the image of the figure was caused due to the subject's own response, or contrary may have a weaker sense that the change was caused due to the subject's own response. Accordingly, when the subject's answer ratio of the answer that the change in the image of the figure was caused due to the subject's own response is aberrant (for example, high or low) in comparison with that of a healthy individual, the subject can be determined to be possibly affected with schizophrenia. The answer ratio can be calculated for each condition by (the number of times of answering that the change in the image of the figure was caused due to the subject's own response)/(the number of times of conducting steps (d) to (g))×100.

Specifically, whether or not the subject's sense of agency is aberrant can be determined as follows. Under the EPA condition, the ratio of the answer "Yes" to the question whether or not the change in the image was caused due to the subject's own response is basically 0%, and the subject's sense of agency can be determined to be aberrant, when the ratio of the answer "Yes" exceeds approximately 10%, approximately 20%, or approximately 30%. Under the AL condition, the subject's sense of agency can be determined to be aberrant, when the ratio remarkably deviates from the sigmoid curve (Figure 9) of healthy individuals. A statistically desirable judgement criteria is such that data from healthy individuals are accumulated, and then, when the ratio falls within ±1 or ±2SD (standard deviation) under each condition, the subject's sense of agency can be determined to be normal, whereas when the ratio deviates from ±1 or ±2SD (standard deviation) under each condition, the subject's sense of agency can be determined to be aberrant.

Whether or not the subject's sense of agency is aberrant can be determined by conducting the sense-of-agency test multiple times on a subject, while changing at random the AL condition or the EPA condition and the time at which the change in the image of the figure occurs,, and considering the obtained results all together. It is also possible to take the results from the subject's passive time perception test into consideration.

Whether or not the sense of agency is aberrant can be judged by using suitable determination means. For example, the determination means may be a computer programed to be capable of judging the subject's second answers (for example, "Yes" or "No"), and calculating the answer ratio thereof, or the like. The determination means can comprise means for judging the second answers (for example, "Yes" or "No") and/or means for calculating the answer ratio.

The methods and device of the present invention are capable of evaluating whether or not a person is affected with schizophrenia, or whether or not a person is prone to be affected with schizophrenia. In addition, the methods and device of the present invention are also capable of evaluating the severity of a patient diagnosed with schizophrenia. Moreover, the methods and device of the present invention can also be used to develop a novel antipsychotic drug for schizophrenia.

### (Examples)

Hereinafter, Examples of the present invention are descried. Example below are described for the better understanding of the claims of the present invention, and are not intended to limit the claims of the present invention.

### (Example 1)

### (Passive Time Perception Test)

A passive time perception test was conducted by using the stimulus application means shown in Figures 1 and 2 and the method shown in Figure 3. The subjects were healthy individuals (n=7) and schizophrenic patients (n=7). The sensory stimuli were a light stimulus and a vibration stimulus, and the time lag was any one of none (simultaneous), 50 msec, or 100 msec. In other words, the following five patterns were prepared. The flowing patterns were conducted at random 50 times in total, with each pattern conducted 10 times:
(i) a tactile stimulus, and a visual stimulus 50 msec after the tactile stimulus;
(ii) a tactile stimulus, and a visual stimulus 100 msec after the tactile stimulus;
(iii) a tactile stimulus and a visual stimulus simultaneously;
(iv) a visual stimulus, and a tactile stimulus 50 msec after the visual stimulus; and
(v) a visual stimulus, and a tactile stimulus 100 msec after the visual stimulus.

Figure 8 shows the results. No difference was present between the healthy individuals and the schizophrenic patients. This means that the subjects of this example had normal passive time perception.

### (Example 2)

### (Sense-of-Agency Test 1)

A sense-of-agency test was conducted by using the method shown in Figure 7. The subjects were healthy individuals (n=35), schizophrenic negative symptom group (n=20), and chronic schizophrenic positive symptom group (n=30).

The image of the figure was a quadrilateral of 5 mm square which was set to move at a speed of 22 mm/second from the bottom of the display. The signal applied to the subjects was a beep sound. The change in the image of the figure was a jump upward by 35 mm. Under the AL condition, the time (delay time) of the change in the image of the figure was 0, 100, 200, 300, 400, 500, 600, 700, 800, 900, or 1000 msec after the pressing of the key. Under the EPA condition, the time of the change in the image of the figure was 100 msec before the beep sound, simultaneously with the beep sound, or 100 msec after the beep sound. The conditions shown in the table below were applied at random 140 times in total, with each condition being applied 10 times. Every time, each subject was questioned "whether he/she felt that his/her pressing of the key caused the figure to make a jump?" and instructed to answer this question with "Yes" or "No."

### [Table 1]

**Table 1. Conditions for Sense-of-Agency Test**

| Conditions | Change in Image (msec) | Number of Runs (times) |
|---|---|---|
| AL Condition | 0 | 10 |
| | 100 | 10 |
| | 200 | 10 |
| | 300 | 10 |
| | 400 | 10 |
| | 500 | 10 |
| | 600 | 10 |
| | 700 | 10 |
| | 800 | 10 |
| | 900 | 10 |
| | 1000 | 10 |
| EPA Condition | -100 | 10 |
| | 0 | 10 |
| | 100 | 10 |

Figure 9 shows the results. Under the AL condition (right graph), the schizophrenic negative symptom group has weaker sense of agency than the healthy individuals even when the delay time between the pressing of the key and the jump of the quadrangle figure is short. In contrast, the schizophrenic positive symptom group has stronger sense of agency than the healthy individuals even when the delay time between the pressing of the key and the jump of the quadrangle figure is long. In addition, it can be seen that, under the EPA condition (left figure), the healthy individuals and the schizophrenic negative symptom group do not have sense of agency, whereas the schizophrenic positive symptom group has strong sense of agency.

### (Example 3)

### (Sense-of-Agency Test 2)

The same sense-of-agency test as that in Example 2 was conducted on patients (n=16) in a prodromal state before onset of schizophrenia. Figure 10 shows an example of the results. The patients in the prodromal state showed a unique pattern in comparison with healthy individuals ((2) in Figure 10). After that, the patients were treated by administration of an antipsychotic drug continuously for approximately 1 month, and the same test was conducted on the patients after the treatment. The treated patients showed a pattern similar to the pattern of the healthy individuals ((3) in Figure 10). This indicates that the patients in the prodromal state before onset of schizophrenia got better by the treatment. These results suggest a potential that the sense-of-agency test of the present invention will greatly contribute also to early diagnosis of schizophrenia and determination to make an early intervention. It is known that the prognosis of schizophrenia is good, when an early intervention can be made. For this reason, the method of the present invention can be an important test.

Subsequently, the same sense-of-agency test as in Example 2 was conducted on patients (n=12) at an acute stage who had actually developed schizophrenia. An example of the results showed chaotic patterns as shown in Figure 11. Based on Figures 9 to 11, the disease stage of schizophrenia (from prodromal stage through onset to chronic stage) can be determined from the standpoint of aberrance in sense of agency. The data obtained by the sense-of-agency test of the present invention can serve as a ground for adopting a suitable therapeutic strategy according to the disease stage.

### (Industrial Applicability)

The method of the present invention is expected to contribute to early detection and early treatment of schizophrenia, and to improve the prognosis of treatment. The method of the present invention would be employed as an essential test for a clinical trial in developing a novel antipsychotic drug against schizophrenia. In addition, the device of the present invention would be regularly installed in hospitals and laboratories all over the world as a testing device for diagnosing schizophrenia in the clinical and research situations.

### (Explanation of the reference numerals)

101. Display that applies visual stimulus
102. Vibrator that applies tactile stimulus
103. Controller that controls visual stimulus and tactile stimulus
104. Subject
201. Computer that controls image of figure
202. Display that displays image of figure moving at constant speed
203. Response button for subject to respond to signal
204. Subject
301. Computer that controls image of figure
302. Display that displays image of figure moving at constant speed
303. Response key for subject to respond to signal
304. Belt portion
305a and 305b. Cursors

## Claims

1. A method for acquiring data for diagnosing schizophrenia, comprising the steps of:
applying a plurality of different sensory stimuli to a subject with any time lag selected from 0 to approximately 1000 msec;
obtaining subject's first answer regarding temporal order judgment of the plurality of different sensory stimuli applied;
determining whether or not subject's passive time perception is normal on the basis of the first answer, wherein the subject's passive time perception can be determined to be normal when a correct-answer ratio of the first answer is high;
displaying an image of a figure moving at a constant speed to the subject whose passive time perception is determined to be normal in the determination step;
giving a signal to the subject;
allowing the subject to respond to the signal,
wherein the image of the figure either
(1) changes due to the subject's response 0 to approximately 2000 msec after the response, or
(2) changes irrespective of the subject's response within approximately 1000 msec before or after the signal; and
obtaining subject's second answer as to whether or not the change in the image of the figure was caused due to the subject's own response;
wherein the subject can be determined to be probably affected with schizophrenia, when an answer ratio of the second answer stating that the change in the image of the figure was caused due to the subject's own response is aberrant in comparison with that of a healthy individual.

2. The method according to claim 1, wherein the plurality of sensory stimuli are a tactile stimulus and a visual stimulus.

3. The method according to claim 2, wherein the tactile stimulus and the visual stimulus are applied in order and with a time lag according to any one of the following (i) to (v):
(i) the tactile stimulus, and the visual stimulus approximately 50 msec after the tactile stimulus;
(ii) the tactile stimulus, and the visual stimulus approximately 100 msec after the tactile stimulus;
(iii) the tactile stimulus and the visual stimulus simultaneously;
(iv) the visual stimulus, and the tactile stimulus approximately 50 msec after the visual stimulus; or
(v) the visual stimulus, and the tactile stimulus approximately 100 msec after the visual stimulus.

4. The method according to claim 2 or 3, wherein the tactile stimulus is a vibration stimulus, and the visual stimulus is a light stimulus.

5. The method according to any one of claims 1 to 4, wherein the change in the image of the figure is disappearance and appearance of the figure.

6. The method according to any one of claims 1 to 5,
wherein the image of the figure
(1) changes due to the subject's response 0 to approximately 1000 msec after the response, or
(2) changes irrespective of the subject's response within approximately 100 msec before and after the signal.

7. The method according to any one of claims 1 to 6,
wherein the step of displaying an image of a figure moving at a constant speed comprises the step of turning on a light of one of two cursors on a belt portion moving at a constant speed, and
wherein the change in the image of the figure is caused by switching the turning-on of the light to turning-on of a light of the other cursor.

8. A device for implementing the method according to any one of claims 1 to 7, comprising:
stimulus application means for applying a plurality of different sensory stimuli to a subject with any time lag selected from 0 to approximately 1000 msec;
first answer acquisition means for obtaining subject's first answer regarding temporal order judgment of the plurality of different sensory stimuli applied;
determination means for determining whether or not subject's passive time perception is normal on the basis of the first answer, wherein the subject's passive time perception can be determined to be normal when a correct-answer ratio of the answer is high;
display means for displaying, to the subject whose passive time perception is determined to be normal by the determination means, an image of a figure that moves at a constant speed and that changes at any one of the following times (1) and (2);
signaling means for giving a signal to the subject;
response means for allowing the subject to respond to the signal:
(1) due to the subject's response 0 to approximately 2000 msec after the response, or
(2) irrespective of the subject's response within approximately 1000 msec before or after the signal; and
second answer acquisition means for obtaining subject's second answer as to whether or not the change in the image of the figure was caused due to the subject's own response,
wherein the subject can be determined to be probably affected with schizophrenia, when an answer ratio of the second answer stating that the change in the image of the figure was caused due to the subject's own response is aberrant in comparison with that of a healthy individual.

9. The device according to claim 8, wherein the plurality of sensory stimuli are a tactile stimulus and a visual stimulus.

10. The device according to claim 9, wherein the tactile stimulus and the visual stimulus are applied in order and with a time lag according to any one of the following (i) to (v):
(i) the tactile stimulus, and the visual stimulus approximately 50 msec after the tactile stimulus;
(ii) the tactile stimulus, and the visual stimulus approximately 100 msec after the tactile stimulus;
(iii) the tactile stimulus and the visual stimulus simultaneously;
(iv) the visual stimulus, and the tactile stimulus approximately 50 msec after the visual stimulus; and
(v) the visual stimulus, and the tactile stimulus approximately 100 msec after the visual stimulus.

11. The device according to claim 9 or 10, wherein the tactile stimulus is a vibration stimulus, and the visual stimulus is a light stimulus.

12. The device according to any one of claims 8 to 11, wherein the change in the image of the figure is disappearance and appearance of the figure.

13. The device according to any one of claims 8 to 12, wherein
the image of the figure
(1) changes due to the subject's response 0 to approximately 1000 msec after the response, or
(2) changes irrespective of the subject's response within approximately 100 msec before and after the signal.

14. The device according to any one of claims 8 to 13,
wherein the display means for displaying an image of a figure comprises a belt portion moving at a constant speed and two cursors joined to the belt portion, and
wherein the image of the figure is displayed by turning on a light of one of the two cursors, and the change in the image of the figure is displayed by switching the turning-on of the light to turning-on of a light of the other cursor.

15. A schizophrenia diagnostic program for implementing the method according to any one of claims 1 to 7, the schizophrenia diagnostic program causing a computer to execute the steps of:
instructing stimulus application means to apply a plurality of different sensory stimuli to a subject with any time lag selected from 0 to approximately 1000 msec;
acquiring, from first answering means, subject's first answer regarding temporal order judgment of the plurality of different sensory stimuli applied;
calculating a correct-answer ratio of the first answer on the basis of the first answer, and outputting a result of a determination that subject's passive time perception is normal, when the correct-answer ratio is high;
causing display means to display an image of a figure moving at a constant speed to the subject whose passive time perception is determined to be normal;
instructing signaling means to give a signal to the subject;
acquiring the subject's response to the signal from response means;
causing the image of the figure on the display means to
(1) change due to the subject's response 0 to approximately 2000 msec after the response, or
(2) change irrespective of the subject's response within approximately 1000 msec before or after the signal;
acquiring, from second answering means, subject's second answer as to whether or not the change in the image of the figure was caused due to the subject's own response; and
calculating the answer ratio of the answer stating that the change in the image of the figure was caused due to the subject's own response based on the second answer, and outputting a result of a determination that the subject is probably affected with schizophrenia, when the answer ratio is aberrant in comparison with that of a healthy individual.

16. A schizophrenia diagnostic program for operating the device according to any one of claims 8 to 14, the schizophrenia diagnostic program causing a computer to execute the steps of:
instructing the stimulus application means to apply a plurality of different sensory stimuli to a subject with any time lag selected from 0 to approximately 1000 msec;
acquiring, from the first answering means, subject's first answer regarding temporal order judgment of the plurality of different sensory stimuli applied;
calculating a correct-answer ratio of the first answer on the basis of the first answer, and outputting a result of a determination that subject's passive time perception is normal, when the correct-answer ratio is high;
causing the display means to display an image of a figure moving at a constant speed to the subject whose passive time perception is determined to be normal;
instructing the signaling means to give a signal to the subject;
acquiring subject's response to the signal from the response means;
causing the image of the figure on the display means to
(1) change due to the subject's response 0 to approximately 2000 msec after the response, or
(2) change irrespective of the subject's response within approximately 1000 msec before or after the signal;
acquiring, from the second answering means, subject's second answer as to whether or not the change in the image of the figure was caused due to the subject's own response; and
calculating an answer ratio of the answer stating that the change in the image of the figure was caused due to the subject's own response based on the second answer, and outputting a result of a determination that the subject is probably affected with schizophrenia, when the answer ratio is aberrant in comparison with that of a healthy individual.

17. A computer-readable recording medium that records the program according to claim 15 or 16.
